# EUROPEAN PATENT SPECIFICATION

(11) **EP 4 352 039 B1**
(45) Date of publication and mention of the grant of the patent: **24.12.2025**
(21) Application number: 22820655.3
(22) Date of filing: 11.05.2022
(51) Int. Cl.: C07C 69/30, A23K 20/158, A61K 31/23, A61P 33/02, C07C 67/03, C11C 3/02, A23K 50/75, A61K 31/20, A61K 31/22, A61K 31/19

(54) **MIXED GLYCEROL ESTER COMPOSITION**
GEMISCHTE GLYCERINESTERZUSAMMENSETZUNG
COMPOSITION D'ESTER DE GLYCÉROL MIXTE

(30) Priority: 09.06.2021 SE 2130157
(43) Date of publication of application: 17.04.2024
(73) Proprietor: Perstorp AB, 284 80 Perstorp (SE)
(72) Inventor: VAESSEN, Stefan, 6721 GX Bennekom (NL); TAWDE, Snehal Nandkumar, 4813NC Breda (NL); SCHWARZER, Conrad Gerard, 3583 Beringen (BE)
(74) Representative: Hoffmann Eitle
(86) International application number: PCT/SE2022/050463
(87) International publication number: WO 2022/260568

(56) References cited:
- EP-A1- 3 229 605
- EP-A1- 3 273 797
- WO-A1-2006/085346
- WO-A1-2015/074767
- WO-A1-2016/093757
- WO-A1-99/66804
- JP-A- H0 556 755
- US-B2- 8 206 772

## Description

### FIELD OF THE INVENTION

The present invention relates to a novel mixed glycerol ester composition comprising glycerol ester molecules comprising both valeric acid moieties and lauric acid moieties in the same molecule. The mixed glycerol ester composition can be useful for mitigating moderate to severe gut health challenges in animals and, in particular, it has shown to be effective for mitigating and/or alleviating coccidiosis in poultry.

The present invention also relates to an animal feed additive comprising said mixed glycerol ester composition and to the use of said animal feed additive for improving the performance, as measured by, for example, the feed conversion ratio, of animals.

### BACKGROUND OF THE INVENTION

It is well known in the art that different short and medium chain fatty acids have multiple positive effects both on animal gut health and performance in general as well as against specific pathogenic bacteria. Utilizing glycerol esters of short and medium chain fatty acids is a technology to achieve the targeted delivery of these acids to the gastrointestinal tract of an animal. There are several advantages associated with delivering the said acids in the form of glycerol esters. Some of the acids, like propionic acid, butyric acid and valeric acid are either corrosive and/or have a pungent, unpleasant odor, which causes handling, safety, manufacturing and transport challenges. Said glycerol esters do not have these issues related to smell or corrosiveness. Delivering short and medium chain fatty acids to an animal in the form of glycerol esters is also a good way to transport the fatty acids to the lower regions of the gastrointestinal tract of an animal. Free short chain fatty acids are likely to be adsorbed and/or disintegrated to a degree in the higher regions of the gastrointestinal canal and will reach the cecum or large intestine in far lower concentrations than intended for an effective dose. Glycerol esters on the other hand will to a large extent reach these regions in a partly dissociated form and a large proportion of the fatty acid will be released and adsorbed in these regions.

Glycerol esters of butyric acid are well known as feed additives because of their ability to improve animal performance and decrease the feed conversion ratio of animals. Further on, glycerol esters of different short and medium chain fatty acids have been shown to inhibit different disease-causing bacteria. For example, it is known from EP3229605 that a glycerol ester composition comprising trivalerin inhibits gram-negative pathogens like *Salmonella.* Further on, EP3273797 discloses that a glycerol ester composition comprising monovalerin inhibits the gram-positive bacteria *Clostridium perfringens,* which causes necrotic enteritis in chicken. It is also known from several sources (for example from Skrivanova et al, Veterinarni Medicina, 51, 2006 (3): 81-88*)* that monolaurin has an inhibitory action on pathogenic, gram-positive bacteria.

The present invention has revealed a new type of glycerol ester composition, comprising an ester wherein both valeric acid and lauric acid moieties are bound to the same glycerol molecule. This unique glycerol ester composition has indicated to be an effective tool for mitigating and alleviating moderate to severe gut health challenges in animals and to increase performance both in non-challenged and challenged chickens. The glycerol ester composition according to the present invention can be useful for mitigating multifactorial gut health syndromes like coccidiosis.

Coccidiosis is a parasitic disease of the intestinal tract of animals caused by protozoa of the genus *Eimeria.* The disease spreads from one animal to another by contact with infected feces or ingestion of infected tissue. Diarrhea, which may become bloody in severe cases, is the primary symptom. Most animals infected with coccidia are asymptomatic, but young or immunocompromised animals may suffer severe symptoms and death.

While coccidia can infect a wide variety of animals, including humans, birds, and livestock, they are usually species-specific and do not spread between species.

Most knowledge on coccidiosis has been obtained from poultry, since coccidiosis remains one of the most prevalent diseases in commercial poultry production with tremendous economic impact. The economic significance of coccidiosis is attributed to decreased animal production (higher feed conversion, growth depression and increased mortality) and the costs involved in treatment and prevention. Blake *et al.* estimated that coccidiosis in chickens cost the UK £99.2 million in 2016 and a global cost of ~ £10.4 billion at 2016 prices, equivalent to £0.16/chicken produced, stressing the urgent need for more efficient strategies to control this parasite *(*Blake et al. Vet Res (2020) 51:115*).*

Tyzzer and colleagues (1929) showed that species of *Eimeria* develop in different regions in the gut of the chicken where, depending on the magnitude of infection, they can cause mild to severe lesions and significant pathology. Depending on the species, magnitude and site of infection, coccidiosis can result in a limited enteritis resulting in fluid loss and malabsorption of nutrients (*E. acervuline and E. mitis*)*,* inflammation of the intestinal wall with pinpoint hemorrhages and sloughing of epithelia (*E. brunetti and E. maxima*)*,* or complete villar destruction resulting in extensive hemorrhage and death (*E. necatrix and E. tenella*)*.*

Today, the main means for control of coccidiosis are vaccination in combination with chemotherapy with anticoccidial drugs or different feed additives like phytochemicals, probiotics and yeast extracts. Anticoccidial drugs can be either synthetic compounds (for example amprolium); polyether antibiotics or ionophores; or mixed products consisting of a synthetic compound and ionophore or two synthetic compounds. However, large-scale and long-term use of anticoccidial drugs has led to the development of resistance against these drugs. In order to minimize the occurrence of resistance, the rotation of various anticoccidial drugs in single and/or shuttle programs is used. Unfortunately, this has not solved the anticoccidial resistance problem. There is an uncertainty about possible upcoming bans restricting the use of anticoccidials as feed additives and an increased pressure both from the society as from human medical perspective to reduce antibiotic use.

Commercial vaccines against coccidiosis were first introduced by Edgar in 1952 and his vaccine (Coccivac) is still widely employed today in various forms. Anticoccidial vaccines are today routine procedure in for example poultry farming. However, only vaccination is in most cases not enough to ensure a satisfactory protection against coccidiosis. A major drawback of live coccidiosis vaccines is their loss of infectivity with time affecting their expiry. Other concerns are their high production costs and managing shortcomings during their application such as dosage errors that may result in insufficient immune response or clinical coccidiosis in case non-attenuated vaccines are used. Subunit vaccines offer possibilities to circumvent the mentioned drawbacks, but unfortunately the immunogenicity of subunit vaccines against *Eimeria* is today not high enough to provide a satisfactory solution to the problem. WO 2016/093757 A1 discloses triglycerides suitable for use in feedstuffs, in which at least 75 wt.% of the triglycerides are glyceryl trivalerate.

US 8206772 B2 relates to feedstuffs comprising fats, in which at least most of the triglycerides present in the fat comprise at least one short chain fatty acid and at least one medium chain fatty acid.

Although maintaining a high standard of hygiene and biosecurity measures could halt the introduction of *Eimeria* to a farm, in practice they do not suffice to prevent coccidiosis outbreaks. Concerns about the use of anticoccidial drugs, widespread parasite drug resistance, the need for more effective vaccines and consumer preferences for "clean" farming, all point to the need for novel, safe and sustainable alternatives to control coccidiosis.

The composition according to the present invention, comprising glycerol ester molecules comprising both valeric acid moieties and lauric acid moieties in the same molecule, has been shown to be an effective tool for mitigating and/or alleviating coccidiosis in poultry. This mixed glycerol ester composition has proven both to reduce *Eimeria* parasites in chicken feces, to reduce lesion scores in challenged birds and to increase performance both in non-challenged birds and in birds challenged with *Eimeria.* The composition according to the present invention has further on indicated to be more effective for increasing performance in chickens challenged with *Eimeria,* compared to a physical mixture of glycerol esters comprising valeric acid and lauric acid respectively.

### DETAILED DESCRIPTION OF THE INVENTION

The search for effective and more sustainable alternatives for mitigating gut health challenges in animals, like coccidiosis, has revealed a glycerol ester composition comprising high levels of a new type of ester molecules, where two different fatty acids are bound to the same glycerol molecule, constituting a mixed glycerol ester.

Mixed glycerol ester molecules can be made either through esterification of glycerol and fatty acids of two different kinds or by reacting glycerol esters of one kind of fatty acid with a free fatty acid of another kind.

Both types of esterification reactions will result in mixtures of fatty acids, glycerol, monoglycerides, diglycerides, triglycerides and mixed glycerol esters of different kinds. The different reaction steps and molar ratios between reactants will determine the composition of the final product. Factors like the availability of water and temperature will also influence the distribution of molecules and internal hydrolyzation as well as re-esterification will most probably occur to some extent. Consequently, it is not trivial to define exactly how the distribution of mixed esters, mono-, di- and tri-esters will be in the final product.

According to one embodiment of the present invention, the mixed glycerol ester composition is produced by reacting valeric acid and monolaurin. Sulfuric acid is preferably used as a catalyst in this reaction and water is continuously removed from the reaction vessel. Excess valeric acid is preferably removed at the end of the reaction.

According to another embodiment of the present invention, the mixed glycerol ester composition is produced by reacting glycerol with valeric acid and lauric acid. This reaction can be run either without any catalyst or with a catalyst. In case a catalyst is used, suitable catalysts are for example sulfuric acid, hydrochloric acid, phosphoric acid, para-toluene sulfonic acid and methyl-sulfonic acid.

The composition according to the present invention comprises 20-60 parts per weight of ester molecules consisting of one glycerol moiety, two valeric acid moieties and one lauric acid moiety. This molecule is abbreviated as *"VVL"* in the scope of the present application and has either structure *(I)* or structure *(II),* illustrated in in *Structure 1:*
The composition according to the present invention also comprises 10-30 parts per weight of ester molecules consisting of one glycerol moiety, one valeric acid moiety and two lauric acid moieties. This molecule is abbreviated as *"VLL"* in the scope of the present application and has either structure *(I)* or structure *(II),* illustrated in *Structure 2:*

In the scope of the present invention, the "VVL" and the "VLL" molecules disclosed above are the main target molecules in the mixed glycerol ester composition. However, as described above there will inevitably also be other molecules present in the composition according to the invention. The other main components of the composition according to the invention are: 10-30 parts per weight of trivalerin, 1-10 parts per weight of trilaurin and 1-10 parts per weight of lauric acid.

In summary, the present invention refers to a mixed glycerol ester composition comprising the following components:
a) 20-60 parts per weight of ester molecules consisting of one glycerol moiety, two valeric acid moieties and one lauric acid moiety
b) 10-30 parts per weight of ester molecules consisting of one glycerol moiety, one valeric acid moiety and two lauric acid moieties
c) 10-30 parts per weight of trivalerin
a) 1-10 parts per weight of trilaurin
d) 1-10 parts per weight of lauric acid

In line with what has been discussed above and, because of the nature of the esterification reactions, there will also be smaller amounts of other molecules present in the final composition. These molecules are for example divalerin, glycerol ester with one valeric acid moiety and one lauric acid moiety and free valeric acid.

According to one embodiment of the present invention the mixed glycerol ester composition additionally comprises a buffering metal salt. Preferably the buffering metal salt is a formate, such as potassium formate or sodium formate.

According to one embodiment of the present invention the mixed glycerol ester composition is adsorbed on an inert carrier, such as a porous silica carrier. This allows the composition to be distributed as a dry product. Distributing the glycerol ester composition adsorbed on a silica carrier contributes to keeping down the water amount in the composition and thereby inhibiting the hydrolyzation of glycerol ester into glycerol and free acid.

The present invention also refers to an animal feed additive comprising a mixed glycerol ester composition as described above.

The feed additive according to the present invention is added to the animal feed at a concentration of between 0.01 to 2.0% by weight, preferably between 0.05-0.1 % by weight.

The feed additive according to the present invention can be added to any commercially available feedstuffs for animals. The feed additive may be incorporated directly into commercially available feeds or fed supplementary to commercially available feeds.

Maintaining a strong gut health is important for all animal, in order to be able to manage different gut health challenges like bacteria, parasites and viruses. Some of the most challenging diseases among animals are so called multifactorial gut health syndromes, like coccidiosis and necrotic enteritis. The mixed glycerol ester composition according to the present invention can be useful for mitigating such multifactorial gut health syndromes in animals and in particular to be effective for mitigating the disease coccidiosis.

According to one embodiment, the present invention refers to the use of said feed additive for mitigating moderate to severe gut health challenges in animals.

According to one embodiment, the present invention refers to the use of said feed additive for mitigating multifactorial gut health syndromes in animals.

Being able to mitigate gut health challenges in a good way will lead to an increased performance. This can be measured as an improved feed conversion ratio (FCR) of the animal.

According to another embodiment, the present invention refers to the use of said feed additive for improving the feed conversion ratio of animals.

According to yet another embodiment, the present invention refers to said mixed glycerol ester composition for use in alleviation and/or mitigation of coccidiosis in animals. Coccidiosis is caused by protozoa of the genus *Eimeria,* with a complex life cycle, affecting mainly the intestinal tract of many species of mammals and birds.

One embodiment of the present invention refers to said mixed glycerol ester composition for use in alleviation and/or mitigation of coccidiosis in chicken, turkey, duck, dog, cattle, rabbit, sheep and/or swine.

Coccidiosis is of great economic significance for the health of farm animals, especially poultry. Most knowledge on coccidiosis has been obtained from poultry, where the disease has been studied most intensively as it is in commercial poultry that it causes the most damage due to the fact that birds are reared together in large numbers and high densities. The economic significance of coccidiosis is attributed to decreased animal production (higher feed conversion, growth depression and increased mortality) and the costs involved in treatment and prevention.

A preferred embodiment of the present invention refers to said composition for use in alleviation and/or mitigation of coccidiosis in chicken, turkey and/or duck.

In a further aspect, the present invention refers to the use of said composition for inhibiting protozoa of the genus *Eimeria* in the gastric tract of animals.

### EMBODIMENT EXAMPLES

The present invention is illustrated in the below Embodiment Examples, which is to be construed as merely illustrative and not limiting in any way.
- *Example 1a discloses a first synthesis route to a composition according to the present invention.*
- *Example 1b discloses a second synthesis route to a composition according to the present invention.*
- *Example 2 illustrates the effect of a composition according to the present invention on performance and fecal oocysts count in non-challenged chickens.*
- *Example 3 illustrates the effect of a composition according to the present invention on performance, lesion score and fecal oocysts count in chickens challenged with Eimeria.*
- *Example 4 illustrates the effect of a composition according to the present invention on performance in chickens challenged with Eimeria; a comparison is made with glycerol ester compositions that are not according to the present invention and with a conventional anti-coccidiosis drug.*
- *Structure 1 illustrates the molecular structure of a mixed ester used in the invention*
- *Structure 2 illustrates the molecular structure of a mixed ester used in the invention*
- *Figures 1-3* *illustrating the results of the experiments performed in Example 3.*
- *Figure 4* *illustrating the results of the experiments performed in Example 4.*

### Example 1a: A first synthesis route to a composition according to the invention

7.0 kg monolaurin, 5.2 kg valeric acid and 0.8 kg heptane was added to a 20 liter jacketed glass reactor equipped with a stirrer. The solution was heated to 150°C using an external oil bath. The reactor temperature was controlled by adding/removing heptane. After 4 hours, 9.8 g sulfuric acid (catalyst) was added to the solution and after 8 hours additionally 0.7 kg valeric acid was added. The synthesis was continued until no more water could be boiled off.

The surplus of valeric acid and heptane was evaporated from the synthesis solution. The synthesis solution was then washed with a 2% sodium hydroxide solution, 2 molar equivalents compared to added sulfuric acid was added of the sodium hydroxide. The solution was then heated to 50° C and stirred for 30 minutes. The stirrer was stopped, and the phases got time to separate. The salt that was created in the wash dropped to the bottom of the reactor and the organic phase was pumped out from the top of the reactor. The solid salt-phase was washed out from the reactor and the organic phase was then added back.

Vacuum was applied with a vacuum pump, to remove any water remaining in the product. The product was taken out through the bottom valve and stored in a 10 liters container. The molar ratio valeric acid : monolaurin in this experiment was 2:1.

The product was analyzed with GC-FID and the composition of the final product is shown below in *Table 1a.* This composition is referred to as *"Mixed glycerol ester VL"* below.

**Table 1a. Mixed glycerol ester VL**

| Compound abbreviation | Explanation | Area % |
|---|---|---|
| VVL | Glycerol ester with one lauric acid residue and two valeric acid residues | 44 |
| VLL | Glycerol ester with two lauric acid residues and on valeric acid residue | 22 |
| GTV | Trivalerin | 23 |
| GTL | Glycerol ester with three lauric acid residues | 2.5 |
| GDV | Divalerin | 0.4 |
| VL | Glycerol ester with one lauric acid residue and one valeric acid residue | 0.6 |
| Lauric acid | | 6.5 |
| Valeric acid | | 0.4 |

### Example 1b: A second synthesis route a composition according to the invention

2.0 kg glycerol, 3.327 kg valeric acid, 4.35 kg of lauric acid and 0.7 kg heptane (used as azeotropic fluid) was added to a 20 liter jacketed glass reactor equipped with a stirrer. The solution was heated to 150°C using an external oil bath. During that heating 9.8 g of sulfuric acid (catalyst) was added once the temperature reached 60°C. The reactor temperature was controlled by adding/removing heptane. After removal of most of the ester water, which took about 3 hours, an additional amount of 3.327 kg of valeric acid was added. The synthesis was continued until no more water could be boiled off which took about another 3 hours.

The surplus of valeric acid and heptane was evaporated from the synthesis solution. The synthesis solution was then washed with 20.3 gram of sodium carbonate in water solution (concentration 10 gram sodium carbonate / liter of water). The solution was then heated to 50° C and stirred for 30 minutes. The stirrer was stopped, and the phases got time to separate. The salt that was created in the wash dropped to the bottom of the reactor and the organic phase was pumped out from the top of the reactor. The solid salt-phase was washed out from the reactor and the organic phase was then added back.

Vacuum was applied with a vacuum pump, to remove any water remaining in the product. The product was taken out through the bottom valve and stored in a 10 liters container. The molar ratio valeric acid : monolaurin in this experiment was 2:1.

The product was analyzed with GC-FID and the composition of the final product is shown below in *Table 1b.* This composition is referred to as *"Mixed glycerol ester VL"* below.

**Table 1b. Mixed glycerol ester VL**

| Compound abbreviation | Explanation | Area % |
|---|---|---|
| VVL | Glycerol ester with one lauric acid residue and two valeric acid residues | 39 |
| VLL | Glycerol ester with two lauric acid residues and on valeric acid residue | 18 |
| GTV | Trivalerin | 25 |
| GTL | Glycerol ester with three lauric acid residues | 2.4 |
| GDV | Divalerin | 2.6 |
| VL | Glycerol ester with one lauric acid residue and one valeric acid residue | 2.9 |
| GDL | Dilaurate | 0.7 |
| Lauric acid | | 8.1 |
| Valeric acid | | 1.3 |

During further lab experimentation it proved possible to reduce the amount of lauric acid from the 8.1 area% above to around and below 4 area% without too much elaboration. In one experiment where an amount of sodium carbonate above equimolar to the sulfur catalyst was used and the amount of free lauric acid in the mixed glycerol ester at 0.1 area% was achieved.

### Example 2: Effect of a composition according to the invention on performance and fecal oocysts count in non-challenged chickens

The experiment was carried out at a research center in the Netherlands. In this experiment, 320 day-old, male Ross 308 broiler chickens from a commercial hatchery were divided into 16 pens, two groups * 8 replicates * 20 animals per pen. The study was continued for 35 days. On day 17, all birds received a Paracox-5 vaccination (in order to simulate real-life conditions).

The two groups were fed the same basal feed, but one of the groups received feed with an addition of 0,1% by weight (1 kg/ton) of the test formulation, which was the composition produced in *Example 1 (Mixed glycerol ester VL)* with a silica carrier (at a weight ratio of *2:1, Mixed glycerol ester VL* : silica).

Weight gain and feed consumption were measured each day for all birds and the average daily weight gain, the average daily feed consumption and the feed conversion ratio (FCR) for day 0-35 were calculated for the control group and the treated group respectively. The results can be seen in *Table* 2 below.

**Table 2.**

| **Group** | **Control** | **Treated** |
|---|---|---|
| **Average feed consumption (g)** | 102,073 | 100,278 |
| **Average daily weight gain (g)** | 67,0 | 67,1 |
| **FCR** | 1,523 | 1,493 |

The average improvement in FCR with the composition according to the invention was three points. The p-value was 0.05 (compared to the control group), which shows that the improvement in FCR was statistically significant.

Samples of droppings were collected from each pen on day 35. Each sample was examined for the number of oocycsts of species *E. maxima* per gram fecal material. The results are shown below in *Table 3.*

**Table 3.**

| **Group** | **Average oocyst count *E. Maxima*** |
|---|---|
| Control | 887,5 |
| Treated | 400 |

It can be seen in Table 3 that treatment with the composition according to the invention gives a more than 50% reduction of *E. maxima* oocysts in feces, compared to the control group.

### Exmple 3: Effect of a composition according to the invention on performance, lesion score and fecal oocysts count in chickens challenged with Eimeria

The experiment was carried out at a research center in the US. In this experiment 320 day-old, male Cobb 500 birds from a commercial hatchery were divided into 40 pens, 5 groups * 8 replicates * 8 animals per pen. The study was continued for 20 days. All birds received routine vaccination (HVT/ SB1, no coccidia vaccination). Birds were weighed on Days 0, 14 and 20. On day 14 birds in the infected treatments received the coccidia inoculum diluted to a 1 ml volume (p.o.). The coccidia inoculum consisted of mixed cultures of three species of *Eimeria.* The species were *E. acervulina* (100,000 oocysts/ bird), *E*. *maxima* (50,000 oocysts/ bird), *and E. tenella.* (75,000 oocysts/ bird). The coccidia inoculum was administered by oral gavage to the birds in the infected treatments. On Day 14 of the study each bird in the noninfected treatment received 1 ml of distilled water by oral pipette (p.o.).

The appropriate feed treatment commenced at placement Day 0 (day of hatch). An unmedicated commercial type chicken starter compounded with feedstuffs commonly used in the United States was formulated. This ration (in mash form) was fed *ad libitum* from the date of chicken arrival until termination of the study. Three different experimental treatment feeds were prepared from this basal starter feed. The experimental feed additive tested was the composition produced in *Example 1 (Mixed glycerol ester VL),* added at three different concentrations. The five different experimental groups are described in *Table* 4 below (% are % by weight). The concentrations in *Table* 4 refer to the ready formulations, consisting of *Mixed glycerol ester VL* with a silica carrier (at a weight ratio of 2:1, *Mixed glycerol ester VL* : silica).

Feed was weighed in on Day 0 and non-consumed feed weighed on Days 14 and 20. The feed conversion ratios (FCRs) for days 0-20 and days 14-20 (after challenge with *Eimeria*) were calculated for the different treatment groups respectively.

**Table 4.**

| **Group** | **Description** |
|---|---|
| 1 | No additive, no Cocci |
| 2 | No additive, Cocci |
| 3 | Mixed glycerol ester VL 0,05%, Cocci |
| 4 | Mixed glycerol ester VL 0,1%, Cocci |
| 5 | Mixed glycerol ester VL 0,2%, Cocci |

### Oocysts per Gram Fecal Material

From all cages, Day 19 to Day 20 samples of the fresh feces (mixture of intestinal and cecal droppings) passed during this period were collected. Feces collected from each cage were thoroughly mixed and prepared for fecal floatation. Each sample was examined for the number of oocycsts by species per gram fecal material.

### Lesion Scoring

On Day 20, five birds were humanely euthanized and then lesion scored by cage. The Johnson and Reid, 1970 method of coccidiosis lesion scoring was used to score the infected region(s) of the intestine.

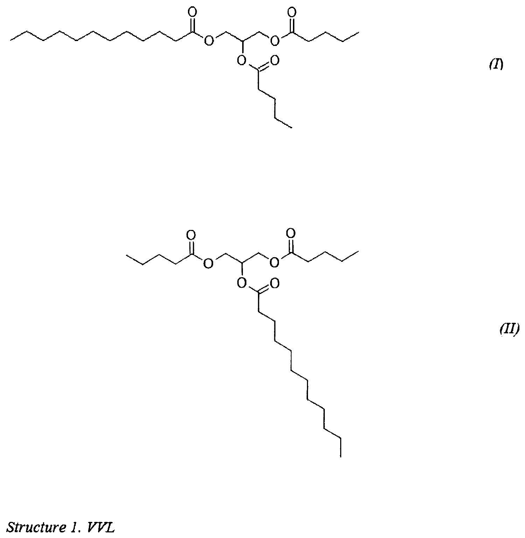

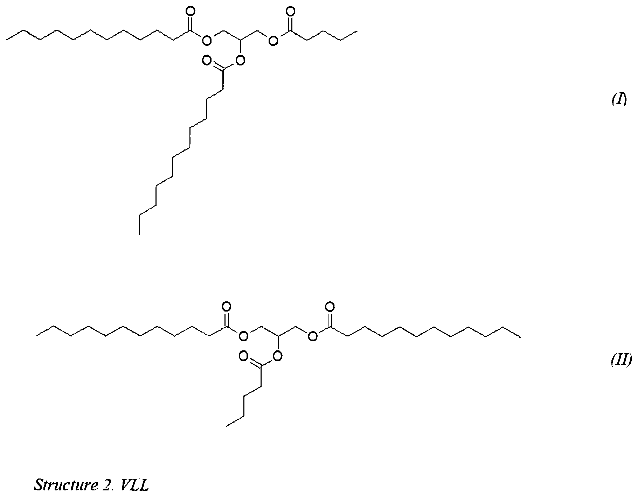

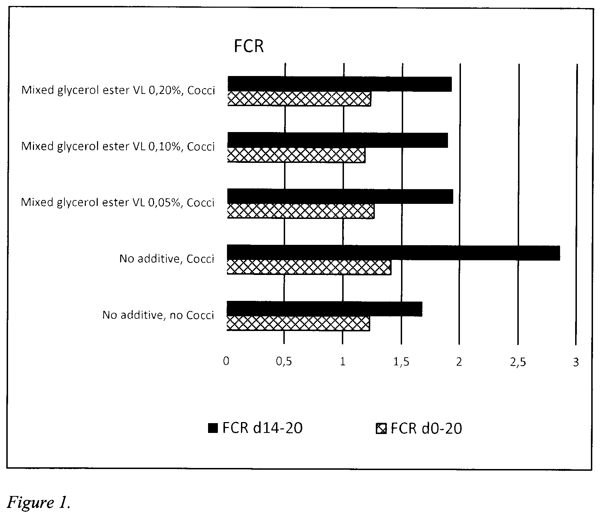

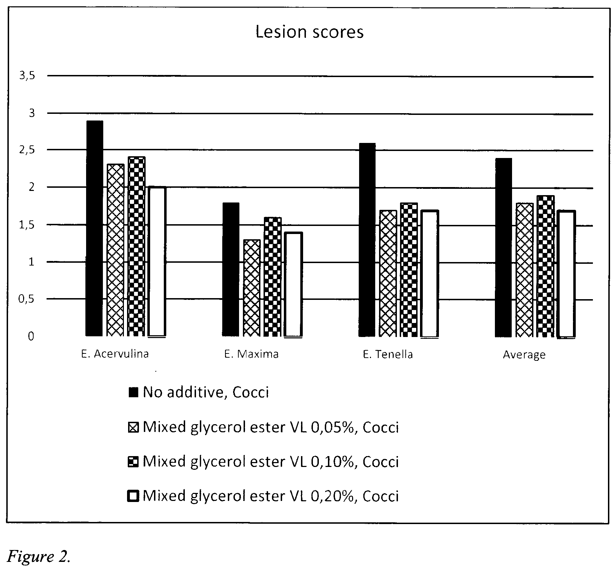

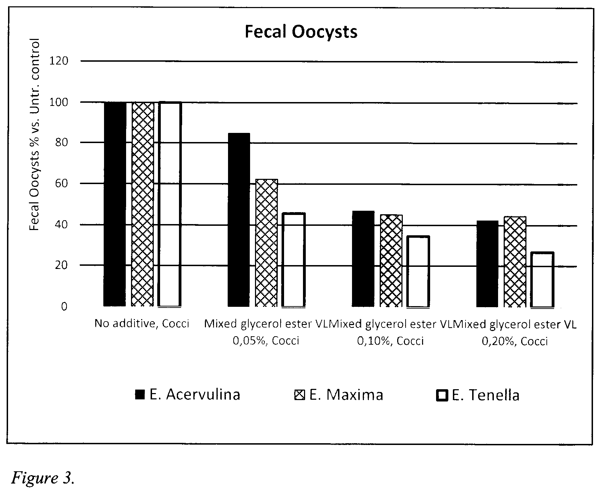

The results of Experiment 3 are shown in Figures 1-3:
The results show that the *Mixed glycerol ester VL* improved performance after an *Eimeria* infection, reduced gut lesions and fecal oocysts for all three species of *Eimeria* that were tested.

### Example 4: Effect of a composition according to the present invention on performance in chickens challenged with Eimeria - comparison with glycerol ester compositions that are not according to the present invention and with a conventional anti-coccidiosis drug.

The experiment was carried out at a research center in the UK. In this experiment, 756 Ross 308 male chickens from a commercial hatchery were divided into seven treatment groups T1-T7 (as outlined in *Table 5*) * nine pens per treatment *12 birds per pen. The study was continues for 20 days. Birds were weighed on days 0, 7, 13 and 20. On day 14 birds in treatment groups T2-T7 were infected with a mixed *Eimeria* inoculum via oral gavage using a syringe. The coccidia inoculum consisted of mixed cultures of *Eimeria,* including at least the *E. tenella, E. maxima* and *E. acervulina* species.

The seven treatment diets were offered from day 0 of the study. The same base diet was fed to the birds throughout the study period and the test items were added to the diets at various inclusion levels, according to *Table 5.* Birds in T1 served as the untreated, uninfected control group and birds in T2 served as untreated, infective control group. Birds in T3 received a commercial coccidiostat reference treatment (*Maxiban*)*.* Birds in T4 received treatment with a mixed glycerol ester composition according to the invention (*Mixed glycerol ester VL, from Example 1)* at an inclusion level of 1.00 g/kg (*Mixed glycerol ester VL* with a silica carrier at a weight ratio of 2:1, *Mixed glycerol ester VL* : silica). Birds in T5, T6 and T7 received treatments with different glycerol ester compositions that were not according to the invention. T5 is a glycerol ester composition comprising mainly trivalerin, T6 is a glycerol ester composition comprising mainly monolaurin and T7 is a physical mixture of trivalerin and monolaurin. The glycerol ester compositions in groups T5, T6 and T7 were included at levels to match the valeric acid and/or lauric acid content added to the feed in treatment group T4. The valeric acid and/or lauric acid content in respective treatment groups were verified by feed analysis after feed preparation. Test formulations T5-T7 were all glycerol ester compositions with silica, at a weight ratio of 2:1, glycerol ester composition : silica.

**Table 5.**

| **Treatment** | **Challenge** | **Test item** | **Inclusion level (g/kg of feed)** |
|---|---|---|---|
| T1 | No | None | N/A |
| T2 | *Eimeria* mix | None | N/A |
| T3 | *Eimeria* mix | Maxiban | 0,50 |
| T4 | *Eimeria* mix | Mixed glycerol ester VL | 1,00 |
| T5 | *Eimeria* mix | GTV - Trivalerin | 1,07 |
| T6 | *Eimeria* mix | ML - Monolaurin | 0,67 |
| T7 | *Eimeria* mix | GTVML - physical mixture of GTV and ML | 0,87 |

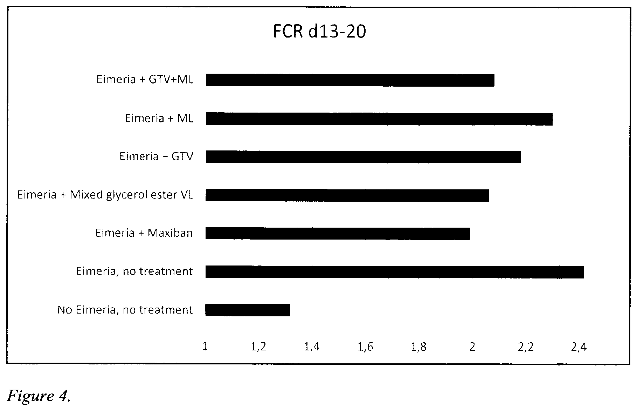

Weight and feed intake were measured (per cage) at days 0, 13 and 20. The feed conversion ratio (FCR) was calculated for the time interval 13-20 days (after challenge with *Eimeria*), for each treatment group and is shown in *Table* 6 and *Figure 4**.*

**Table 6.**

| **Treatment** | **FCR d13-20** |
|---|---|
| T1 | 1.32 |
| T2 | 2.42 |
| T3 | 1.99 |
| T4 | 2.06 |
| T5 | 2.18 |
| T6 | 2.30 |
| T7 | 2.08 |

*Table 6* and *Figure 4* indicates that the glycerol ester composition according to the invention (treatment T4) gives a lower FCR than a physical mixture of trivalerin and monolaurin (treatment T7), after the birds have been challenged with *Eimeria.*

## Claims

1. A mixed glycerol ester composition **characterized in, that** it comprises:
a) 20-60 parts per weight of ester molecules consisting of one glycerol moiety, two valeric acid moieties and one lauric acid moiety
b) 10-30 parts per weight of ester molecules consisting of one glycerol moiety, one valeric acid moiety and two lauric acid moieties
c) 10-30 parts per weight of trivalerin
d) 1-10 parts per weight of trilaurin, and
e) 1-10 parts per weight of lauric acid.

2. An animal feed additive comprising a mixed glycerol ester composition according to claim 1.

3. Use of an animal feed additive according to claim 2 **characterized in, that** said animal feed additive is added to the feed at a concentration of between 0.01-2.0 % by weight.

4. Use of an animal feed additive according to claim 3 **characterized in, that** said animal feed additive is added to the feed at a concentration of between 0.05-0.1 % by weight.

5. A mixed glycerol ester composition according to claim 1, for use in alleviation and/or mitigation of coccidiosis in animals.

6. A mixed glycerol ester composition for use according to claim 5 **characterized in, that** said animals are chicken, turkey, duck, dogs, cattle, rabbit, sheep and/or swine.

7. A mixed glycerol ester composition for use according to claim 6 **characterized in, that** said animals are chicken, turkey and/or duck.

8. A mixed glycerol ester composition according to claim 1, for use in inhibiting protozoa of the genus Eimeria in the gastric tract of animals.

## Patentansprüche

1. Gemischte Glycerinesterzusammensetzung, **dadurch gekennzeichnet, dass** sie umfasst:
a) 20-60 Gewichtsteile Estermoleküle, bestehend aus einer Glycerin-Gruppe, zwei Valeriansäure-Gruppen und einer Laurinsäure-Gruppe,
b) 10-30 Gewichtsteile Estermoleküle, bestehend aus einer Glycerin-Gruppe, einer Valeriansäure-Gruppe und zwei Laurinsäure-Gruppen,
c) 10-30 Gewichtsteile Trivalerin,
d) 1-10 Gewichtsteile Trilaurin und
e) 1-10 Gewichtsteile Laurinsäure.

2. Tierfuttermittelzusatz, umfassend eine gemischte Glycerinesterzusammensetzung gemäß Anspruch 1.

3. Verwendung eines Tierfuttermittelzusatzes gemäß Anspruch 2, **dadurch gekennzeichnet, dass** der Tierfuttermittelzusatz dem Futter in einer Konzentration zwischen 0,01-2,0 Gew.-% zugesetzt wird.

4. Verwendung eines Tierfuttermittelzusatzes gemäß Anspruch 3, **dadurch gekennzeichnet, dass** der Tierfuttermittelzusatz dem Futter in einer Konzentration zwischen 0,05-0,1 Gew.-% zugesetzt wird.

5. Gemischte Glycerinesterzusammensetzung gemäß Anspruch 1 zur Verwendung zur Linderung und/oder Milderung von Kokzidiose bei Tieren.

6. Gemischte Glycerinesterzusammensetzung zur Verwendung gemäß Anspruch 5, **dadurch gekennzeichnet, dass** die Tiere Hühner, Truthähne, Enten, Hunde, Rinder, Kaninchen, Schafe und/oder Schweine sind.

7. Gemischte Glycerinesterzusammensetzung zur Verwendung gemäß Anspruch 6, **dadurch gekennzeichnet, dass** die Tiere Hühner, Truthähne und/oder Enten sind.

8. Gemischte Glycerinesterzusammensetzung gemäß Anspruch 1 zur Verwendung bei der Hemmung von Protozoen der Gattung Eimeria im Magen-Darm-Trakt von Tieren.

## Revendications

1. Composition d'ester de glycérol mixte **caractérisée en ce qu'**elle comprend :
a) entre 20 et 60 parties en poids de molécules d'ester consistant en un fragment de glycérol, deux fragments d'acide valérique et un fragment d'acide laurique
b) entre 10 et 30 parties en poids de molécules d'ester consistant en un fragment de glycérol, un fragment d'acide valérique et deux fragments d'acide laurique
c) entre 10 et 30 parties en poids de trivalérine
d) entre 1 et 10 parties en poids de trilaurine, et
e) entre 1 et 10 parties en poids d'acide laurique.

2. Additif alimentaire pour animaux comprenant une composition d'ester de glycérol mixte selon la revendication 1.

3. Utilisation d'un additif alimentaire pour animaux selon la revendication 2, **caractérisée en ce que** ledit additif alimentaire pour animaux est ajouté à l'alimentation selon une concentration comprise entre 0,01 % en poids et 2,0 % en poids.

4. Utilisation d'un additif alimentaire pour animaux selon la revendication 3, **caractérisée en ce que** ledit additif alimentaire pour animaux est ajouté à l'alimentation selon une concentration comprise entre 0,05 % en poids et 0,1 % en poids.

5. Composition d'ester de glycérol mixte selon la revendication 1, destinée à être utilisée dans le soulagement et/ou l'atténuation de la coccidiose chez des animaux.

6. Composition d'ester de glycérol mixte destinée à être utilisée selon la revendication 5, **caractérisée en ce que** lesdits animaux sont des poulets, des dindes, des canards, des chiens, des bœufs, des lapins, des moutons et/ou des porcs.

7. Composition d'ester de glycérol mixte destinée à être utilisée selon la revendication 6, **caractérisée en ce que** lesdits animaux sont des poulets, des dindes et/ou des canards.

8. Composition d'ester de glycérol mixte selon la revendication 1, destinée à être utilisée dans l'inhibition de protozoaires du genre Eimeria dans le tractus intestinal d'animaux.
